# EUROPÄISCHE PATENTSCHRIFT

(11) **EP 2 432 756 B1**
(45) Veröffentlichungstag und Bekanntmachung des Hinweises auf die Patenterteilung: **21.12.2016**
(21) Anmeldenummer: 10723029.4
(22) Anmeldetag: 19.05.2010
(51) Int. Cl.: C07C 29/149, C07C 29/17, C07C 31/125

(54) **VERFAHREN ZUR HERSTELLUNG VON FETTALKOHOLEN DURCH HYDRIERUNG VON FETTSÄURETRIGLYCERIDEN AN EINEM KUPFERHALTIGEN HETEROGENEN KATALYSATOR**
PROCESS FOR PRODUCING FATTY ALCOHOLS BY HYDROGENATION OF FATTY ACID TRIGLYCERIDES ON A COPPER-CONTAINING HETEROGENEOUS CATALYST
PROCÉDÉ DE PRODUCTION D'ALCOOLS GRAS PAR HYDROGÉNATION DE TRIGLYCÉRIDES D'ACIDE GRAS EN PRÉSENCE D'UN CATALYSEUR HÉTÉROGÈNE CONTENANT DU CUIVRE

(30) Priorität: 20.05.2009 EP 09160709
(43) Veröffentlichungstag der Anmeldung: 28.03.2012
(73) Patentinhaber: BASF SE, 67056 Ludwigshafen am Rhein (DE)
(72) Erfinder: MIRK, Daniela, 67454 Haßloch (DE); PROCHAZKA, Roman, 68163 Mannheim (DE); HENKELMANN, Jochem, 68165 Mannheim (DE); HÖLZLE, Markus, 67281 Kirchheim (DE); HERZBERG, Daniela, 1950 Sion (DE)
(74) Vertreter: BASF IP Association
(86) Internationale Anmeldenummer: PCT/EP2010/056859
(87) Internationale Veröffentlichungsnummer: WO 2010/133619

(56) Entgegenhaltungen:
- DE-A1- 10 313 702
- US-A- 5 475 160
- US-A1- 2004 133 049

## Beschreibung

Die vorliegende Erfindung betrifft ein Verfahren zur Herstellung von Fettalkoholen, bei dem man einen Strom bereitstellt, der wenigstes ein Fettsäuretriglycerid enthält und diesen Strom einer Hydrierung in Gegenwart eines kupferhaltigen, heterogenen Katalysators unterzieht.

Fettalkohole sind wichtige Zwischenprodukte für eine Vielzahl chemischer Erzeugnisse, wie z. B. Tenside und kosmetische Produkte. Ihre Herstellung kann beispielsweise durch Hydrierung von Fettsäuremethylestern erfolgen, die aus fett- oder ölhaltigen Ausgangsprodukten durch Umesterung der enthaltenen Triglyceride erhältlich sind. Dabei fällt als weiteres Wertprodukt Glycerin an, welches beispielsweise einer Hydrierung unter Erhalt von 1,2-Propandiol unterzogen werden kann.

Es ist bekannt, die Hydrierung von Glycerin zur Herstellung von 1,2-Propandiol in Gegenwart heterogener, kupferhaltiger Katalysatoren durchzuführen. Solche Verfahren sind z. B. in WO 2007/099161, WO 2009/027500, WO 2009/027501 und WO 2009/027502 beschrieben.

Es ist weiterhin bekannt, Fettalkohole durch Direkthydrierung von triglyceridhaltigen Ausgangsstoffen, z. B. von natürlichen Ölen und Fetten, herzustellen.

Die DE-AS 1 154 448 beschreibt ein Verfahren zur Herstellung höhermolekularer ein- und mehrwertiger Alkohole durch katalytische Hydrierung von Fetten, Fettsäuren und Fettsäureestern unter Verwendung von tablettierten Kupfer-Zink-, Kupfer-Chrom-, Kupfer-Mangan-, Kupfer-Zink-Chrom-, Kupfer-Mangan-Chrom-, Kupfer-Kadmium-, Kupfer-Kadmium-Chrom- und Kupfer-Kadmium-Mangan-Katalysatoren.

Die EP 0 063 427 A2 beschreibt ein Verfahren zur selektiven Hydrierung von ungesättigten Fettsäurederivaten in Gegenwart von Ammoniak und einem Katalysator, der mindestens ein Metall, ausgewählt unter Pd, Pt, Ir und Rh, aufweist.

Die EP 0 254 189 A2 beschreibt ein Verfahren zur Direkthydrierung von Glyceridölen in Gegenwart von Katalysatoren, die 30 bis 40 Gew.-% Kupfer, 23 bis 30 Gew.-% Chrom, 1 bis 7 Gew.-% Barium, jeweils bezogen auf oxidische Katalysatormasse, sowie gewünschtenfalls weitere Übergangsmetalle in Form ihrer Oxide enthalten.

Die DE 37 08 430 A1 beschreibt ein Verfahren zur Direkthydrierung von Butterfett in Gegenwart von Katalysatoren, die 30 bis 40 Gew.-% Kupfer, 23 bis 30 Gew.-% Chrom, 1 bis 10 Gew.-% Mangan, 1 bis 10 Gew.-% Silicium und 1 bis 7 Gew.-% Barium, jeweils bezogen auf oxidische Katalysatormasse, sowie gewünschtenfalls weitere Übergangsmetalle in Form ihrer Oxide enthalten.

Die DE 41 29 622 A1 beschreibt ein Verfahren zur Herstellung von ungesättigten Fettalkoholen durch Hydrierung an einem Zink-Chrom-Katalysator vom Spinell-Typ.

Die DE 198 43 798 A1 beschreibt ein Verfahren zur Herstellung von Fettalkoholen durch Hydrierung von Fettsäuren oder Fettsäureestern an einem Katalysatorfestbett, bei dem nicht umgesetzter Wasserstoff zurückgewonnen und in die Hydrierung zurückgeführt wird und der Reaktor durch Begasen mit nicht aufgeheiztem Wasserstoff gekühlt wird.

Die WO 01/43873 A2 beschreibt oxidische Zink-Aluminium-Katalysatoren für die Herstellung von ungesättigten Fettalkoholen durch Hydrierung von ungesättigten Fettsäuren, Fettsäureniedrigalkylestern oder Fettsäureglyceriden.

Die US 2004/0133049 A1 beschreibt ein Verfahren zur Herstellung von Fettalkoholen aus Fettsäuren, Fettsäureestern und natürlich vorkommenden Triglyceriden in einem Festbettreaktor. Als geeignete Hydrierkatalysatoren werden u. a. oxidische Kupfer-Aluminium-Katalysatoren beschrieben.

Die WO 96/14280 beschreibt ein Verfahren zur direkten Hydrierung von Carbonsäureestern zu Fettalkoholen in Gegenwart eines Katalysators, der eine Kupferverbindung, eine Zinkverbindung und wenigstens eine Verbindung, die ausgewählt ist unter Verbindungen von Aluminium, Zirkon, Magnesium, Seltenerdmetallen und Mischungen davon, umfasst.

Die DE 42 42 466 A1 beschreibt ein Verfahren zur Herstellung von Fettalkoholen, bei dem man natürliche Fette und Öle in Gegenwart von gegebenenfalls kalzinierten Kupfer-Zink-Katalysatoren bei einem hohen Wasserstoffdruck und hoher Temperatur in einem kontinuierlich betriebenen Rohrbündelreaktor hydriert.

Die US 5475160 A beschreibt ein Verfahren zur direkten Hydrierung von Carbonsäureestern in einer vorwiegend flüssigen Phase unter Anwendung eines Katalysators, der eine Kupferverbindung, eine Zinkverbindung und wenigstens eine Verbindung, ausgewählt aus der aus Aluminium, Zirkon, Magnesium, einem Seltenerdmetall und deren Gemischen bestehenden Gruppe, umfasst.

Die DE 10313702 A1 beschreibt ein Verfahren zur Hydrierung einer mindestens eine Carbonylgruppe aufweisenden organischen Verbindung, bei dem die organische Verbindung in Anwesenheit von Wasserstoff mit einem Formkörper in Kontakt gebracht wird, der herstellbar ist gemäss einem Verfahren, in dem (i) ein oxidisches Material, umfassend Kupferoxid, Aluminiumoxid und mindestens eins der Oxide des Lanthans, Wolframs, Molybdäns, Titans oder Zirkoniums, bereitgestellt wird, (ii) dem oxidischen Material pulverförmiges metallisches Kupfer, Kupferblättchen, pulverförmiger Zement, Graphit oder ein Gemisch davon zugegeben wird und (iii) das aus (ii) resultierende Gemisch zu einem Formkörper verformt wird.

Die US 2004/133049 A1 beschreibt ein Verfahren zur kontinuierlichen Hydrierung von Fettsäuren und Fettsäureestern unter Verwendung von Festbettkatalysatoren, bei dem nicht umgesetzter Wasserstoff ohne Wiedererwärmen in den Hydrierschritt zurückgegeben und die Hydrierungsreaktion unter erhöhten Drücken durchgeführt wird.

Der vorliegenden Erfindung liegt die Aufgabe zu Grunde, ein verbessertes Verfahren zur Herstellung von Fettalkoholen zur Verfügung zu stellen. So sind die bekannten Verfahren noch verbesserungswürdig hinsichtlich einer möglichst vollständigen Hydrierung der Fettsäuretriglyceride bei gleichzeitig guter Selektivität bezüglich 1,2-Propandiol als weiterem Wertprodukt. Das bereitgestellte Verfahren soll sich insbesondere sowohl zur Verarbeitung von Fettsäuretriglyceridströmen aus biogenen Quellen als auch von technisch anfallenden Fettsäuretriglyceridströmen eignen.

Überraschenderweise wurde nun gefunden, dass sich kupferhaltige, heterogene Katalysatoren, wobei die katalytisch aktive Komponente des Katalysators zusätzlich Aluminium und Lanthan enthält, besonders vorteilhaft zur einstufigen Hydrierung von Fettsäuretriglyceridströmen unter Erhalt von Fettalkoholen eignen. Sie ermöglichen insbesondere eine im Wesentlichen vollständige einstufige Hydrierung. Vorteilhafterweise zeichnen sich diese Katalysatoren auch durch eine hohe Selektivität gegenüber 1,2-Propandiol als weiterem Wertprodukt der Hydrierung aus.

Gegenstand der Erfindung ist daher ein Verfahren zur Herstellung von Fettalkoholen mit 6 bis 40 Kohlenstoffatomen, bei dem man
a) einen Strom bereitstellt, der wenigstes ein Fettsäuretriglycerid enthält, das im Wesentlichen aus Glycerinestern höherer Fettsäuren mit 6 bis 40 Kohlenstoffatomen besteht,
b) den Fettsäuretriglycerid enthaltenden Strom einer Hydrierung in Gegenwart eines kupferhaltigen, heterogenen Katalysators unterzieht, wobei zur Hydrierung in Schritt b) ein Katalysator eingesetzt wird, der aus Kupfer, Aluminium und Lanthan besteht, wobei die Katalysatoren die Metalle in oxidischer Form, reduzierter Form oder einer Kombination davon enthalten können,
c) aus dem in Schritt b) erhaltenen Hydrierungsprodukt wenigstens eine fettalkoholhaltige Fraktion isoliert.

Der Begriff Fettalkohol bezeichnet im Rahmen der Erfindung gesättigte und ungesättigte Alkohole mit 6 bis 40, vorzugsweise 8 bis 30, insbesondere 10 bis 28 Kohlenstoffatomen. Dabei handelt es sich insbesondere um lineare Alkohole.

In einer speziellen Ausführung handelt es sich um gesättigte Fettalkohole oder Fettalkoholgemische, die überwiegend gesättigte Fettalkohole (d. h. zu mindestens 80 %) aufweisen.

Das erfindungsgemäße Verfahren ermöglicht eine einstufige (d. h. direkte) Hydrierung eines fettsäuretriglyceridhaltigen Ausgangsmaterials zu einem fettalkoholhaltigen Produkt. Unter einstufig wird dabei verstanden, dass das fettsäuretriglyceridhaltige Ausgangsmaterial ohne vorherige Umesterung der Fettsäuretriglyceride, z. B. zu Fettsäuremethylestern, zur Hydrierung eingesetzt wird. Das erfindungsgemäße Verfahren ermöglicht auch die Hydrierung eines fettsäuretriglyceridhaltigen Ausgangsmaterials ohne vorherige Umsetzung zur Erhöhung des Gehalts an freien Fettsäuren. Dabei kann die Hydrierung nach dem erfindungsgemäßen Verfahren jedoch in wenigstens zwei hintereinander geschalteten Hydrierreaktoren erfolgen. In dieser Ausgestaltung wird jedoch in der Regel auf die Isolierung teilhydrierter Zwischenprodukte verzichtet.

### Schritt a)

Zur Bereitstellung des fettsäuretriglyceridhaltigen Stroms eignen sich prinzipiell alle verfügbaren biogenen öl- und/oder fetthaltigen Ausgangsgemische. Öle und Fette sind allgemein feste, halbfeste oder flüssige Fettsäuretriglyceride, insbesondere aus pflanzlichen und tierischen Quellen, die chemisch im Wesentlichen aus Glycerinestern höherer Fettsäuren bestehen. Erfindungsgemäß verwendete höhere Fettsäuren sind gesättigte oder ein- oder mehrfach ungesättigte Fettsäuren mit 6 bis 40, besonders bevorzugt 8 bis 30, insbesondere 10 bis 28 Kohlenstoffatomen. Dazu zählen z. B. n-Nonansäure, n-Decansäure, n-Undecansäure, n-Tridecansäure, Myristinsäure, Pentadecansäure, Palmitinsäure, Margarinsäure, Nonadecansäure, Arachinsäure, Behensäure, Lignocerinsäure, Cerotinsäure, Melissinsäure, Palmitoleinsäure, Oleinsäure, Linolsäure, Linolensäure, Stearinsäure, Elaostearinsäure, etc.

Pflanzliche Fette und Öle basieren im Wesentlichen auf Fettsäuren mit gerader Kohlenstoffatomanzahl, wohingegen tierische Fette und Öle auch Fettsäuren mit ungerader Kohlenstoffatomanzahl in als Triglyceridester gebundener Form enthalten können. Die in pflanzlichen Fetten und Ölen vorkommenden ungesättigten Fettsäuren liegen in der cis-Form vor, während tierische Fettsäuren häufig trans-konfiguriert sind.

Zur Bereitstellung des fettsäuretriglyceridhaltigen Stroms in Schritt a) können prinzipiell gebrauchte oder ungebrauchte, ungereinigte oder gereinigte pflanzliche, tierische oder technische Öle oder Fette oder Gemische davon eingesetzt werden. Diese können Anteile weiterer Inhaltsstoffe, z. B. freie Fettsäuren, enthalten. Der Anteil freier Fettsäuren beträgt im Allgemeinen 0 % bis 20 %, z. B. 0,1 bis 15 %, bezogen auf das Gesamtgewicht des in Schritt a) bereit gestellten fettsäuretriglyceridhaltigen Stroms.

Zur Bereitstellung des fettsäuretriglyceridhaltigen Stroms in Schritt a) können freie Fettsäuren gewünschtenfalls teilweise oder vollständig entfernt werden. Salze dieser Fettsäuren (z. B. die Alkalisalze) können zuvor durch Ansäuern mit einer starken Säure, z. B. HCl, in die freie Säure überführt werden. Die Abtrennung der freien Fettsäuren gelingt z. B. durch Zentrifugieren.

Zur Bereitstellung des fettsäuretriglyceridhaltigen Stroms in Schritt a) geeignete ungebrauchte Fette und Öle sind fett- oder ölhaltige Komponenten, die nach ihrer Gewinnung aus den entsprechenden pflanzlichen oder tierischen Ausgangsmaterialien noch keinem anderen Zweck zugeführt wurden und die daher nur Inhaltsstoffe aufweisen, die aus den Ausgangsmaterialien stammen bzw. die mit der Gewinnung aus den Ausgangsmaterialien im Zusammenhang stehen. Gewünschtenfalls können aus diesen Ausgangsmaterialien andere Inhaltsstoffe als Fettsäuretriglyceride (und gegebenenfalls freie Fettsäuren) vor dem Einsatz zur Hydrierung in Schritt b) zumindest teilweise entfernt werden.

Zur Bereitstellung des fettsäuretriglyceridhaltigen Stroms in Schritt a) geeignete gebrauchte Fette und Öle sind fett- und/oder ölhaltige Komponenten, die nach ihrer Gewinnung aus entsprechenden biogenen Ausgangsmaterialien zunächst zu anderen Zwecken, z. B. zu technischen Zwecken oder Zwecken der Nahrungsmittelherstellung, verwendet wurden und die infolge dieser Verwendung chemisch modifiziert oder unmodifiziert sind oder zusätzliche Inhaltsstoffe, die insbesondere im Zusammenhang mit dieser Verwendung stehen, aufweisen können. Diese können gewünschtenfalls zur Bereitstellung des fettsäuretriglyceridhaltigen Stroms zumindest teilweise entfernt werden.

Zur Aufreinigung und/oder Anreicherung können die ungebrauchten oder gebrauchten Fette oder Öle einer Entfernung von ungewünschten Inhaltsstoffen, wie Lecithinen, Kohlenhydraten, Proteinen, Ölschlamm, Wasser, etc. unterzogen werden.

Pflanzliche Öle und Fette sind solche, die zum überwiegenden Teil aus pflanzlichen Ausgangsmaterialien wie Samen, Wurzeln, Blättern oder anderen geeigneten Pflanzenteilen stammen. Tierische Fette oder Öle stammen zum überwiegenden Teil aus tierischen Ausgangsmaterialien, wie tierischen Organen, Geweben oder anderen Körperteilen oder Körperflüssigkeiten wie Milch. Technische Öle und Fette sind solche, die insbesondere aus tierischen oder pflanzlichen Ausgangsmaterialien gewonnen und für technische Zwecke aufbereitet wurden. Die erfindungsgemäß eingesetzten gebrauchten oder ungebrauchten, ungereinigten oder gereinigten Öle und/oder Fette sind insbesondere ausgewählt aus der Gruppe, bestehend aus Soapstock, Brown Grease, Yellow Grease, technischem Talg, technischem Schmalz, Frittierölen, Tierfett, Speisetalg, pflanzlichen Rohölen, tierischen Rohölen oder -fetten oder Gemischen davon.

Unter "Soapstock" wird ein bei der Verarbeitung von pflanzlichen Ölen anfallendes Nebenprodukt verstanden, insbesondere ein Nebenprodukt von Speiseöl-Raffinerien auf der Basis von Soja-, Rüb- oder Sonnenblumenöl. Soapstock weist einen Anteil von freien Fettsäuren von etwa 50 % bis 80 % auf.

Unter "Brown Grease" wird ein tierfetthaltiges Abfallprodukt verstanden, das einen Anteil von freien Fettsäuren von über 15 % bis 40 % aufweist. "Yellow Grease" enthält etwa 5 % bis 15 % freie Fettsäuren.

Unter "technischem Talg" und "technischem Schmalz" werden tierische Fette verstanden, die für technische Zwecke hergestellt und nach dem Trocken- oder Nassschmelzverfahren beispielsweise aus Schlachtabfällen gewonnen werden. Technische Talge werden nach ihrer Säurezahl bewertet und gehandelt, wobei der Gehalt an freien Fettsäuren je nach Herkunft z. B. zwischen 1 und 20 Gew.-%, wie beispielsweise im Bereich von 1 bis 15 Gew.-%, liegt. Der Gehalt an freien Fettsäuren kann jedoch auch mehr als 20 Gew.-% betragen.

Zu den "Tierfetten" gehören insbesondere bei der Verwertung von Geflügel-, Rinder-, Schweine-, Fisch- und Meeressäuger-Körpern abfallende fetthaltige Produkte, beispielsweise Solarstearin, ein fester Rückstand, der nach dem Auspressen von Schmalzöl aus Schweineschmalz verbleibt.

Die Bereitstellung des fettsäuretriglyceridhaltigen Stroms in Schritt a) erfolgt vorzugsweise aus pflanzlichen Rohölen als Ausgangsmaterial. Dabei kann man von ungereinigten pflanzlichen Rohölen ausgehen, d. h. von flüssigen oder festen Zusammensetzungen, die aus pflanzlichen Ausgangsmaterialien z. B. durch Pressen gewonnen werden, wobei sie keine andere Behandlung erfahren haben als Absetzen in allgemein üblichen Zeiträumen und Abschleudern oder Filtrieren, bei dem zur Trennung des Öls von festen Bestandteilen nur mechanische Kräfte wie Schwerkraft, Fliehkraft oder Druck eingesetzt werden. Solche ungereinigten pflanzlichen Rohöle können auch durch Extraktion gewonnene pflanzliche Öle sein, wenn sich deren Eigenschaften von den entsprechenden, mittels Pressen gewonnenen pflanzlichen Ölen nicht oder nur unwesentlich unterscheiden. Der Anteil freier Fettsäuren in ungereinigten pflanzlichen Fetten und Ölen ist unterschiedlich und liegt z. B. bei etwa 0 bis 20 %, wie z. B. 0,1 bis 15 %.

Selbstverständlich können die pflanzlichen Öle vor ihrem Einsatz zur Hydrierung in Schritt b) einem oder mehreren Aufarbeitungsschritten unterzogen werden, wie sie im Folgenden noch genauer beschrieben sind. So können auch gereinigte pflanzliche Öle, beispielsweise Raffinate oder Halbraffinate, der vorstehend genannten pflanzlichen Öle als Ausgangsmaterialien eingesetzt werden.

Vorzugsweise wird zur Bereitstellung des fettsäuretriglyceridhaltigen Stroms in Schritt a) ein pflanzliches Öl bzw. Fett eingesetzt, das vorzugsweise ausgewählt ist unter Rapsöl, Palmöl, Rüböl, Sojaöl, Sonnenblumenöl, Maiskeimöl, Baumwollsaatöl, Palmkern- und Kokosfett und Mischungen davon. Besonders bevorzugt wird Rapsöl oder ein rapsölhaltiges Gemisch eingesetzt.

Geeignet zur Bereitstellung des fettsäuretriglyceridhaltigen Stroms in Schritt a) ist auch tierisches Öl bzw. Fett, das vorzugsweise ausgewählt ist unter Milchfett, Wollfett, Rindertalg, Schweineschmalz, Fischölen, Fischtran, etc. und Mischungen davon.

Der in Schritt a) bereit gestellte fettsäuretriglyceridhaltigen Strom weist vorzugsweise einen Wassergehalt von höchstens 30 Gew.-%, bevorzugt von höchstens 20 Gew.-%, auf. In einer speziellen Ausführungsform wird in Schritt a) ein fettsäuretriglyceridhaltiger Strom bereit gestellt, der im Wesentlichen wasserfrei ist. Unter "im Wesentlichen wasserfrei" wird im Rahmen der vorliegenden Erfindung ein Wassergehalt von höchstens 3 Gew.-%, besonders bevorzugt von höchstens 1 Gew.-%, verstanden. Der Einsatz von fettsäuretriglyceridhaltigen Strömen mit einem Wassergehalt im Bereich bis 30 Gew.-%, insbesondere bis 20 Gew.-%, ermöglicht in dem zur Hydrierung in Schritt b) eingesetzten Temperatur- und Druckbereich die Herstellung von Fettalkoholen und von 1,2-Propandiol mit hohen Ausbeuten und mit hoher Selektivität.

Die fettsäuretriglyceridhaltigen Ströme können anstelle von oder zusätzlich zu Wasser wenigstens ein organisches Lösungsmittel aufweisen. Bevorzugt weisen die in Schritt a) bereitgestellten fettsäuretriglyceridhaltigen Ströme einen Gesamtlösungsmittelgehalt von höchstens 20 Gew.-%, besonders bevorzugt höchstens 15 Gew.-%, insbesondere höchstens 10 Gew.-% und speziell höchstens 5 Gew.-% auf. Werden Lösungsmittelgemische eingesetzt, die Wasser und wenigstens ein wassermischbares organisches Lösungsmittel enthalten, so beträgt der Anteil des organischen Lösungsmittels vorzugsweise höchstens 50 Gew.-%, besonders bevorzugt höchstens 20 Gew.-%, bezogen auf das Gesamtgewicht des Lösungsmittels. Bevorzugte organische Lösungsmittel sind C₁-C₄-Alkanole, wie Methanol, Ethanol, n-Propanol, Isopropanol, n-Butanol, tert.-Butanol, Polyole und Mono- und Dialkylether davon, cyclische Ether, wie Dioxan und Tetrahydrofuran, etc. Geeignete Lösungsmittel sind auch aromatische Kohlenwasserstoffe, wie Benzol, Toluol oder die Xylole. Bevorzugt als organische Lösungsmittel sind C₁-C₄-Alkanole, insbesondere Methanol und/oder Ethanol und deren Gemische mit Wasser. Bevorzugt weisen die in Schritt a) bereitgestellten und zur Hydrierung in Schritt b) eingesetzten fettsäuretriglyceridhaltigen Ströme jedoch keine organischen Lösungsmittel auf.

Die in Schritt a) bereitgestellten fettsäuretriglyceridhaltigen Ströme können wenigstens einem Aufarbeitungsschritt unterzogen werden. Dazu zählt z. B. wenigstens ein Reinigungsschritt zur Entfernung unerwünschter Komponenten. Dazu zählt weiterhin eine Verringerung des Gehalts an Wasser und/oder, soweit vorhanden, organischer Lösungsmittel.

Die Bereitstellung des fettsäuretriglyceridhaltigen Stroms in Schritt a) umfasst in einer speziellen Ausführungsform wenigstens einen Reinigungsschritt. Dazu kann ein fett- und/oder ölhaltiges Ausgangsgemisch wenigstens einem üblicherweise verwendeten Reinigungsverfahren für Fette und Öle, wie Klären, Filtration, Behandlung mit Bleicherden oder Behandlung mit Säuren oder Alkali zur Abtrennung störender Verunreinigungen wie Proteine, Phosphatide und Schleimstoffe sowie einer Kombination von wenigstens zwei dieser Reinigungsschritte unterzogen werden.

Je nach Herkunft können die fettsäuretriglyceridhaltigen Ströme noch anorganische Salze als unerwünschte Komponente enthalten. Diese können nach bekannten Aufarbeitungsverfahren entfernt werden. Dazu eignet sich insbesondere eine thermische Aufarbeitung (z. B. unter Einsatz eines Verdampfers beispielsweise eines Sambay-Verdampfers).

Je nach Herkunft können die fettsäuretriglyceridhaltigen Ströme auch Katalysatorgifte, d. h. Komponenten, welche die Hydrierung beeinträchtigen, indem sie den Hydrierungskatalysator deaktivieren, enthalten. Hierzu zählen z. B. stickstoffhaltige Verbindungen, wie Amine, und schwefelhaltige Verbindungen, wie Schwefelsäure, Schwefelwasserstoff, Thioalkohole, Thioether, z. B. Dimethylsulfid und Dimethyldisulfid, Kohlenoxidsulfid, Aminosäuren, z. B. Schwefel- und zusätzliche Stickstoffgruppen enthaltende Aminosäuren, Fettsäuren und deren Salz, etc. Zu den Katalysatorgiften zählen weiterhin Halogenverbindungen, Spuren von üblichen Extraktionsmitteln, z. B. Acetonitril oder N-Methylpyrrolidon, etc. sowie gegebenenfalls organische Phosphor- und Arsenverbindungen.

Zur Aufarbeitung der fettsäuretriglyceridhaltigen Ströme in Schritt a) kann z. B. eine thermische Aufarbeitung, bevorzugt eine Destillation, eine Adsorption, ein lonenaustausch, ein Membrantrennverfahren, eine Kristallisation, eine Extraktion oder eine Kombination aus zwei oder mehreren dieser Verfahren zum Einsatz kommen. Membrantrennverfahren unter Einsatz von Membranen definierter Porengrößen eignen sich speziell zur Verringerung des Wassergehalts und/oder zur Salzentfernung. Unter Kristallisation wird auch das partielle Ausfrieren der fettsäuretriglyceridhaltigen Ströme an gekühlten Oberflächen verstanden. Somit lassen sich Verunreinigungen entfernen, die sich in der festen Phase anreichern.

In einer ersten Ausführung wird der fettsäuretriglyceridhaltige Strom in Schritt a) einer Destillation zur Verringerung des Wassergehalts und/oder zur Entfernung von Komponenten, welche die katalytische Hydrierung beeinträchtigen, unterzogen. Diese kann prinzipiell nach üblichen, dem Fachmann bekannten Destillationsverfahren erfolgen. Geeignete Vorrichtungen zur destillativen Aufarbeitung umfassen Destillationskolonnen, wie Bodenkolonnen, die mit Glocken, Siebplatten, Siebböden, Packungen, Füllkörpern, Ventilen, Seitenabzügen, etc. ausgerüstet sein können, Verdampfer, wie Dünnschichtverdampfer, Fallfilmverdampfer, Zwangsumlaufverdampfer, Sambay-Verdampfer, etc. und Kombinationen davon.

Geeignete Trennverfahren werden in den folgenden Dokumenten beschrieben: Sattler, Klaus: Thermische Trennverfahren, 3. Auflage, Wiley VCH, 2001; Schlünder E. U., Thurner F.: Destillation, Absorption, Extraktion, Springer Verlag, 1995; Mersmann, Alfons: Thermische Verfahrenstechnik, Springer Verlag, 1980; Grassmann P., Widmer F.: Einführung in die thermische Verfahrenstechnik, de Gruyter, 1997; Weiß S., Militzer K.-E., Gramlich K.: Thermische Verfahrenstechnik, Dt. Verlag für Grundstoffindustrie, Leipzig, Stuttgart, 1993. Auf diese Dokumente wird hier Bezug genommen.

In einer weiteren Ausführung wird der fettsäuretriglyceridhaltige Strom in Schritt a) zur Verringerung des Gehalts an schwefelhaltigen Verbindungen, speziell schwefelhaltigen aromatischen Verbindungen, einer katalytischen Entschwefelung, gegebenenfalls in Gegenwart von Wasserstoff, unterzogen. Geeignete Entschwefelungsmittel umfassen eine Metallkomponente, wobei das Metall vorzugsweise ausgewählt ist unter Metallen der Gruppen 6, 7, 8, 9, 10, 11 und 12 des Periodensystems. Vorzugsweise werden die Metalle ausgewählt unter Mo, Ni, Cu, Ag, Zn und Kombinationen davon. Geeignete weitere Komponenten der Entschwefelungsmittel sind Dotierstoffe. Die Metallkomponente kann in oxidischer Form, reduzierter Form oder einer Mischung, die oxidierte und reduzierte Komponenten enthält, eingesetzt werden. Die aktive Komponente der Entschwefelungsmittel (Metallkomponente(n) und gegebenenfalls Dotierstoff(e)) können auf einen Träger aufgebracht werden. Geeignete Träger sind prinzipiell die im Folgenden genannten Adsorbentien und Katalysatorträger. Vorzugsweise ist das Trägermaterial ausgewählt unter Aktivkohle, Graphit, Ruß, Al₂O₃, SiO₂ TiO₂ ZrO₂, SiC, Silikaten, Zeolithen, Tonerden (z. B. Bentonite) und Kombinationen davon. Das Aufbringen wenigstens einer Metallkomponente und gegebenenfalls weiterer Komponenten auf ein Trägermaterial kann nach bekannten Verfahren erfolgen, z. B. durch (Co)-Präzipitation oder Imprägnieren. Die Entschwefelungsmittel können als Formkörper eingesetzt werden, z. B. in Form von gepressten Zylindern, Tabletten, Pastillen, Wagenrädern, Ringen, Sternen oder Strangpresslingen, wie Vollsträngen, polylobären Strängen, Hohlsträngen und Wabenkörpern oder anderen geometrischen Körpern. Ungeträgerte Entschwefelungsmittel können nach üblichen Verfahren geformt werden, z. B. durch Extrudieren, Tablettieren, etc. Die Form geträgerter Entschwefelungsmittel wird durch die Form des Trägers bestimmt.

Zur katalytischen Entschwefelung wird vorzugsweise ein Entschwefelungsmittel eingesetzt, das Kupfer und Zink in einem Atomverhältnis von 1 : 0,3 bis 1:10, vorzugsweise 1 : 0,5 bis 1 : 3, insbesondere 1 : 0,7 bis 1 : 1,5, enthält. Bevorzugt wird ein Entschwefelungsmittel eingesetzt, das 35 bis 45 Gew.-% Kupferoxid, 35 bis 45 Gew.-% Zinkoxid und 10 bis 30 Gew.-% Aluminiumoxid enthält. In einer speziellen Ausführungsform handelt es sich bei dem Entschwefelungsmittel um eine zum Einsatz als Hydrierungskatalysator in Schritt b) befähigte Komponente. Diesbezüglich wird auf die folgende Offenbarung zu Hydrierkatalysatoren und Verfahren zu ihrer Herstellung Bezug genommen.

In einer Ausgestaltung dieser Verfahrensvariante werden die fettsäuretriglyceridhaltigen Ströme in wenigstens einer Entschwefelungszone mit dem Entschwefelungsmittel in Kontakt gebracht und anschließend in wenigstens einer Reaktionszone hydriert.

Es versteht sich für den Fachmann, dass die konkrete Ausgestaltung und Anordnung der Entschwefelungs- und Reaktionszone(n) in jeder bekannten Weise erfolgen kann. Es ist möglich, die Entschwefelungs- und Reaktionszone(n) räumlich voneinander getrennt anzuordnen, d. h. durch die apparative Ausgestaltung baulich voneinander zu separieren oder auch in einer oder mehreren gemeinsamen Entschwefelungs-/-Hydrierzone(n) zu verwirklichen.

Das Kupfer-Zink-Entschwefelungsmittel kann z. B. durch ein übliches Fällungs- oder Copräzipitationsverfahren erhalten und in oxidierter als auch in reduzierter Form eingesetzt werden.

In einer besonderen Ausführungsform enthält das Kupfer-Zink-Entschwefelungsmittel mindestens Kupfer, Zink und Aluminium, wobei das Kupfer: Zink : Aluminium-Atomverhältnis im Bereich von 1 : 0,3 : 0,05 bis 1 : 10 : 2, vorzugsweise bei 1 : 0,6 : 0,3 bis 1 : 3 : 1 und insbesondere bei 1 : 0,7 : 0,5 bis 1 : 1,5 : 0,9 liegt.

Zur Überführung in die reduzierte Form ist es möglich, das Entschwefelungsmittel einer Wasserstoffreduktion zu unterwerfen. Diese wird bei etwa 150 bis 350 °C, vorzugsweise bei etwa 150 bis 250 °C, in Gegenwart von Wasserstoff durchgeführt, wobei der Wasserstoff durch ein Inertgas, wie z. B. Stickstoff, Argon, Methan, insbesondere Stickstoff, verdünnt wird, so dass der Wasserstoffgehalt 10 Vol.-% oder weniger, vorzugsweise 6 Vol.-% oder weniger, insbesondere 0,5 bis 4 Vol.-%, beträgt. Das so erhaltene Kupfer-Zink-Entschwefelungsmittel ("reduzierte Form") kann in dieser Form in die Entschwefelung eingesetzt werden.

In einer Ausführungsform wird die Entschwefelung des fettsäuretriglyceridhaltigen Stroms an dem Kupfer-Zink-Entschwefelungsmittel in oxidierter Form ohne Zusatz von Wasserstoff durchgeführt.

In einer weiteren Ausführungsform wird die Entschwefelung des fettsäuretriglyceridhaltigen Stroms an dem Kupfer-Zink-Entschwefelungsmittel in oxidierter Form in Gegenwart von Wasserstoff durchgeführt.

In einer weiteren Ausführungsform wird die Entschwefelung des fettsäuretriglyceridhaltigen Stroms an dem Kupfer-Zink-Entschwefelungsmittel in reduzierter Form ohne Zusatz von Wasserstoff durchgeführt.

In einer weiteren Ausführungsform wird die Entschwefelung des fettsäuretriglyceridhaltigen Stroms an dem Kupfer-Zink-Entschwefelungsmittel in reduzierter Form in Gegenwart von Wasserstoff durchgeführt.

Üblicherweise wird die Entschwefelung in einem Temperaturbereich von 40 bis 200 °C, besonders bei 50 bis 180 °C, insbesondere bei 60 bis 160 °C, vorzugsweise bei 70 bis 120 °C, bei einem Druck von 1 bis 40 bar, besonders bei 1 bis 32 bar, vorzugsweise bei 1,5 bis 5 bar, insbesondere bei 2,0 bis 4,5 bar, durchgeführt. Die Entschwefelung kann in Gegenwart von Inertgasen, wie z. B. Stickstoff, Argon oder Methan, durchgeführt werden. In der Regel wird jedoch die Entschwefelung ohne Zugabe von Inertgasen durchgeführt.

Üblicherweise setzt man - soweit gewünscht - hierbei Wasserstoff mit einer Reinheit von ≥ 99,8 Vol.-%, insbesondere von ≥ 99,9 Vol.-%, vorzugsweise von ≥ 99,95 Vol.-%, ein. Diese Reinheitsgrade gelten analog für den Wasserstoff, der bei den gegebenenfalls durchgeführten Aktivierungen der Katalysatoren verwendet wird.

Üblicherweise liegt das Gewichtsverhältnis von fettsäuretriglyceridhaltigem Strom zu Wasserstoff im Bereich von 40000 : 1 bis 1000 : 1, besonders im Bereich von 38000 : 1 bis 5000 : 1, insbesondere im Bereich von 37000 : 1 bis 15000 : 1, vorzugsweise im Bereich von 36000 : 1 bis 25000 : 1, im Speziellen im Bereich von 35000 : 1 bis 30000:1.

Der so entschwefelte fettsäuretriglyceridhaltige Strom hat im Allgemeinen einen Gehalt von schwefelhaltigen Verunreinigungen, speziell von aromatischen Schwefelverbindungen, von höchstens 70 ppb, vorzugsweise von höchstens 50 ppb, und der Gesamtschwefelgehalt liegt bei insgesamt ≤ 200 ppb, vorzugsweise ≤ 150 ppb, insbesondere ≤ 100 ppb.

Die voranstehend beschriebenen Entschwefelungsmittel ermöglichen es auch, Chlor, Arsen und/oder Phosphor bzw. entsprechende chlor-, arsen- und/oder phosphorhaltige Verbindungen aus dem fettsäuretriglyceridhaltigen zu reduzieren oder zu entfernen.

In einer weiteren Ausführung wird der fettsäuretriglyceridhaltige Strom in Schritt a) zur Entfernung von Komponenten, welche die katalytische Hydrierung beeinträchtigen, mit wenigstens einem Adsorptionsmittel in Kontakt gebracht.

Die Adsorptionsmittel haben vorzugsweise eine spezifische Oberfläche, bestimmt nach BET, im Bereich von 10 bis 2000 m²/g, besonders bevorzugt im Bereich von 10 bis 1500 m²/g, insbesondere im Bereich von 10 bis 400 m²/g, speziell im Bereich von 60 bis 250 m²/g.

Geeignete Adsorptionsmittel sind z. B. aktive Aluminiumoxide. Ihre Herstellung erfolgt z. B. ausgehend von Aluminiumhydroxid, welches durch übliche Fällungsverfahren aus Aluminiumsalzlösungen erhältlich ist. Für das erfindungsgemäße Verfahren geeignete aktive Aluminiumoxide sind auch ausgehend von Aluminiumhydroxid-Gelen erhältlich. Zur Herstellung solcher Gele kann z. B. gefälltes Aluminiumhydroxid nach üblichen Aufarbeitungsschritten, wie Filtern, Waschen und Trocknen, aktiviert und anschließend gegebenenfalls vermahlen oder agglomeriert werden. Gewünschtenfalls kann man das resultierende Aluminiumoxid anschließend noch einem Formgebungsverfahren, wie Extrusion, Granulation, Tablettierung etc. unterwerfen. Als Adsorptionsmittel eignen sich vorzugsweise die Selexsorb TM-Typen der Firma Alcoa.

Geeignete Adsorptionsmittel sind weiterhin aluminiumoxidhaltige Feststoffe. Hierzu zählen z. B. die so genannten Tonerden, die als Hauptbestandteil ebenfalls Aluminiumoxide aufweisen.

Des Weiteren geeignete Adsorptionsmittel sind Aluminiumphosphate.

Weiterhin geeignete Adsorptionsmittel sind Siliciumdioxide, die z. B. durch Entwässerung und Aktivierung von Kieselgelen erhältlich sind. Ein weiteres Verfahren zur Herstellung von Siliciumdioxid ist die Flammhydrolyse von Siliciumtetrachlorid, wobei durch geeignete Variationen der Reaktionsparameter, wie z. B. der stöchiometrischen Zusammensetzung des Eduktgemisches und der Temperatur, die gewünschten Oberflächeneigenschaften des resultierenden Siliciumdioxids in weiten Bereichen variiert werden können.

Weiterhin geeignete Adsorptionsmittel sind Kieselgure, die ebenfalls als Hauptbestandteil Siliciumdioxide aufweisen. Dazu zählt z. B. die aus Kieselsedimenten gewonnene Diatomeenerde.

Weiterhin geeignete Adsorptionsmittel sind Titandioxide und Zirkoniumdioxide, wie sie z. B. in Römpp, Chemie-Lexikon, 9. Aufl. (Paperback), Bd. 6, S. 4629f. und S. 5156f. und der dort zitierten Literatur beschrieben sind. Hierauf wird hier in vollem Umfang Bezug genommen.

Weiterhin geeignete Adsorptionsmittel sind Phosphate, insbesondere kondensierte Phosphate, wie z. B. Schmelz- oder Glühphosphate, die eine große aktive Oberfläche aufweisen. Geeignete Phosphate sind z. B. in Römpp, Chemie-Lexikon, 9. Aufl. (Paperback), Bd. 4, S. 3376f. und der dort zitierten Literatur beschrieben. Hierauf wird hier in vollem Umfang Bezug genommen.

Weiterhin geeignete Adsorptionsmittel sind kohlenstoffhaltige Adsorbentien, bevorzugt Aktivkohle. Unter Aktivkohle versteht man hierbei im Allgemeinen Kohlenstoff mit poröser Struktur und hoher innerer Oberfläche. Zur Herstellung von Aktivkohle werden pflanzliche, tierische und/oder mineralische kohlenstoffhaltige Rohstoffe, z. B. mit Dehydratisierungsmitteln, wie Zinkchlorid oder Phosphorsäure, erhitzt oder durch trockene Destillation verkohlt und anschließend oxidativ aktiviert. Dazu kann man z. B. das verkohlte Material bei erhöhten Temperaturen von etwa 700 bis 1000 °C mit Wasserdampf, Kohlendioxid und/oder Gemischen davon behandeln.

Möglich ist auch ein Einsatz von Ionenaustauschern und/oder Adsorberharzen.

Die Adsorptionsmittel sind vorzugsweise unter Titandioxiden, Zirkoniumdioxiden, Siliciumdioxiden, Kieselgur, Aluminiumoxiden, aluminiumoxidhaltigen Feststoffen, Aluminiumphosphaten, natürlichen und synthetischen Aluminiumsilicaten, Phosphaten, kohlenstoffhaltigen Adsorbentien und Mischungen davon ausgewählt.

Die Adsorptionsmittel weisen im Allgemeinen eine spezifische Oberfläche, bestimmt nach BET, im Bereich von etwa 10 bis 2000 m²/g, insbesondere im Bereich von 10 bis 1500 m²/g und speziell im Bereich von 20 bis 600 m²/g, auf.

Zur adsorptiven Entfernung von unerwünschten Komponenten, insbesondere von Komponenten, welche die katalytische Hydrierung beeinträchtigen, wird der fettsäuretriglyceridhaltige Strom in Schritt a) in einer Adsorptionszone mit wenigstens einem Adsorptionsmittel in Kontakt gebracht.

In einer speziellen Ausführungsform wird ein Adsorptionsmittel eingesetzt, welches wenigstens eine auch zum Einsatz als Hydrierungskatalysator in Schritt b) befähigte Komponente enthält. Auf die im Folgenden genauer beschriebenen Hydrierungskatalysatoren wird hier im vollen Umfang Bezug genommen. Zum Einsatz als Adsorptionsmittel eignen sich auch Kombinationen aus zwei oder mehr als zwei Adsorptionsmitteln. Dabei können sowohl ausschließlich auch als Hydrierungskatalysatoren befähigte Komponenten, ausschließlich nicht als Hydrierungskatalysatoren geeignete Adsorptionsmittel sowie Kombinationen davon eingesetzt werden.

In einer bevorzugten Ausführungsform setzt man als Adsorptionsmittel und als Hydrierungskatalysator die gleiche Komponente ein. Gegebenenfalls setzt man hierbei zusätzlich ein oder mehrere weitere, von dem Hydrierungskatalysator verschiedene herkömmliche Adsorptionsmittel, wie zuvor beschrieben, ein.

In einer Ausgestaltung des Verfahrens werden die fettsäuretriglyceridhaltigen Ströme in wenigstens einer Adsorptionszone mit dem Adsorptionsmittel in Kontakt gebracht und anschließend in wenigstens einer Reaktionszone hydriert.

Es versteht sich für den Fachmann, dass die konkrete Ausgestaltung und Anordnung der Adsorptions- und Reaktionszone(n) in jeder bekannten Weise erfolgen kann. Es ist bevorzugt, die Adsorptions- und Reaktionszone(n) räumlich voneinander getrennt anzuordnen, d. h. durch die apparative Ausgestaltung baulich voneinander zu separieren.

Sofern man unterschiedliche Adsorptionsmittel verwendet, kann man z. B. eine erste Adsorptionszone in einem ersten Reaktor vorsehen, die ein erstes Adsorptionsmittel enthält, und separat, also apparativ getrennt davon, z. B. in einem zweiten Reaktor, eine zweite Adsorptionszone, die ein zweites Adsorptionsmittel enthält. Hierbei kann das erste und/oder das zweite Adsorptionsmittel wenigstens eine zum Einsatz als Hydrierungskatalysators befähigte Komponente enthalten.

In einer weiteren Ausführungsform setzt man ein herkömmliches Adsorptionsmittel zusammen mit einem zur Hydrierung befähigten Adsorptionsmittel in einer einzigen Adsorptionszone ein, z. B. in übereinander geschichteter Form, gemischt in Form einer statistischen Verteilung oder in Form einer Gradientenschüttung. Die Verwendung in gemischter Form erlaubt gegebenenfalls eine bessere Kontrolle der Temperatur. Im Fall einer Gradientenschüttung können lineare und nicht-lineare Gradienten verwendet werden. Es kann hierbei vorteilhaft sein, die Verteilung innerhalb der Schüttung derart vorzunehmen, dass der zu hydrierende fettsäuretriglyceridhaltige Strom zunächst mit dem herkömmlichen Adsorptionsmittel in Kontakt gebracht wird, bevor er mit dem zur Hydrierung befähigten Adsorptionsmittel in Kontakt gebracht wird.

Vorteilhafterweise wird man wenigstens zwei Adsorptionszonen so anordnen, dass der zu hydrierende fettsäuretriglyceridhaltige Strom in der ersten Adsorptionszone mit einem herkömmlichen Adsorptionsmittel in Kontakt gebracht wird und in der zweiten Adsorptionszone mit einem Adsorptionsmittel, das wenigstens eine zum Einsatz als Hydrierungskatalysator befähigte Komponente enthält, in Kontakt gebracht wird.

### Schritt b)

Erfindungsgemäß wird zur Hydrierung in Schritt b) ein heterogener Katalysator eingesetzt, der aus Kupfer, Aluminium und Lanthan besteht.

Bei den in dem erfindungsgemäßen Verfahren eingesetzten heterogenen Hydrierungskatalysatoren kann es sich um Vollkatalysatoren oder geträgerte Katalysatoren handeln. Sie können in Form von einheitlich zusammengesetzten Katalysatoren, imprägnierten Katalysatoren, beschichteten Katalysatoren und Fällungskatalysatoren eingesetzt werden.

Geeignete Katalysatoren können die Metalle in oxidischer Form, reduzierter Form (elementarer Form) oder einer Kombination davon enthalten. Metalle, die in mehr als einer Oxidationsstufe stabil sind, können vollständig in einer der Oxidationsstufen oder in verschiedenen Oxidationsstufen eingesetzt werden.

Eine spezielle Ausführungsform von Katalysatoren, die sich besonders vorteilhaft für einen Einsatz in dem erfindungsgemäßen Verfahren eignen, sind Katalysatoren, die Kupfer in oxidischer Form sowie gegebenenfalls zusätzlich in elementarer Form enthalten. Der in Schritt b) eingesetzte Hydrierkatalysator enthält dann vorzugsweise wenigstens 25 Gew.-%, besonders bevorzugt wenigstens 35 Gew.-%, Kupfer in oxidischer und/oder elementarer Form, bezogen auf das Gesamtgewicht des Katalysators.

Ein häufig angewandtes Verfahren zur Herstellung solcher Katalysatoren besteht in der Tränkung von Trägermaterialien mit Lösungen der Katalysatorkomponenten, die anschließend durch thermische Behandlung, Zersetzung oder Reduktion in den katalytisch aktiven Zustand überführt werden.

Ein weiteres geeignetes Verfahren zur Herstellung von Katalysatoren umfasst die Fällung (Präzipitation) wenigstes einer Katalysatorkomponente. Verschieden Katalysatorkomponenten können nacheinander gefällt werden oder zwei oder mehr als zwei Katalysatorkomponenten können in einer Co-Fällung gefällt werden. So kann zur Herstellung eines Katalysatorformkörpers eine Kupferverbindung, wenigstens eine weitere Metallverbindung und gegebenenfalls wenigstens ein Additiv gefällt und anschließend einer Trocknung, Kalzination und Formung unterzogen werden. Die Fällung kann in Gegenwart eines Trägermaterials durchgeführt werden. Geeignete Ausgangsmaterialien für die Fällung sind Metallsalze und Metallkomplexe. Als Metallverbindungen für die Fällung können prinzipiell alle bekannten Metallsalze eingesetzt werden, die in den zur Auftragung auf den Träger eingesetzten Lösungsmitteln löslich sind. Dazu zählen z. B. Nitrate, Carbonate, Acetate, Oxalate oder Ammoniumkomplexe. In einer bevorzugten Ausführung wird wenigstens ein Metallnitrat eingesetzt. Vorzugsweise wird zur Fällung ein wässriges Medium eingesetzt.

Geeignete wässrige Medien sind Substanzen oder Mischungen, die unter den Verfahrensbedingungen flüssig sind und die wenigstens 10 Gew.-%, besonders bevorzugt wenigstens 30 Gew.-%, insbesondere wenigstens 50 Gew.-%, Wasser enthalten. Der von Wasser verschiedene Anteil ist vorzugsweise ausgewählt unter anorganischen oder organischen Verbindungen, die wenigstens teilweise in Wasser löslich oder wenigstens teilweise mit Wasser mischbar sind. Beispielsweise sind die von Wasser verschiedenen Verbindungen ausgewählt unter organischen Lösungsmitteln, wie C₁-C₂₀-Alkanolen, insbesondere Methanol, Ethanol, n-Propanol, Isopropanol, n-Butanol, sec.-Butanol, tert.-Butanol, Penthanolen und Hexanolen, C₄-C₈-Cycloalkylethern, wie Tetrahydrofuranen, Pyranen, Dioxanen und Trioxanen, C₁-C₁₂-Dialkylethern, wie Dimethylether, Dibutylether und Methylbutylether. Das wässrige Medium enthält vorzugsweise weniger als 40 Gew.-%, besonders bevorzugt weniger als 30 Gew.-% und insbesondere weniger als 20 Gew.-% organische Lösungsmittel. In einer bevorzugten Ausführung des erfindungsgemäßen Verfahrens ist das wässrige Medium im Wesentlichen frei von organischen Lösungsmitteln.

Die Fällung kann durch bekannte Verfahren, z. B. Kühlen einer gesättigten Lösung, Zugabe eines Fällungsmittels, etc. induziert werden. Geeignete Fällungsmittel sind z. B. Säuren, Basen, Reduktionsmittel, etc.

Die Fällung kann durch Zugabe einer Säure oder einer Base zu dem wässrigen Medium induziert werden, das die Kupferverbindung und gegebenenfalls weitere Verbindungen enthält. Geeignete Säuren sind Mineralsäuren, wie HCl, H₂SO₄ und H₃PO₄. Die Base ist vorzugsweise ausgewählt unter Metalloxiden, Metallhydroxiden, insbesondere Alkalimetallhydroxiden, wie Natriumhydroxid und Kaliumhydroxid, Metallcarbonaten, insbesondere Alkalimetall- und Erdalkalimetallcarbonaten, wie Lithiumcarbonat, Natriumcarbonat, Kaliumcarbonat, Magnesiumcarbonat und Calciumcarbonat, Stickstoffbasen, insbesondere Ammoniak sowie primären, sekundären und tertiären Aminen.

Beispiele geeigneter Reduktionsmittel sind Carbonsäuren, wie Ameisensäure, Citronensäure, Milchsäure, Weinsäure und insbesondere Salze von Carbonsäuren, vorzugsweise die Alkalimetall-, Erdalkalimetall-, Ammonium- und C₁-C₁₀-Alkylammoniumsalze, phosphorige oder hypophosphorige Säure, die Salze von phosphoriger oder hypophosphoriger Säure, insbesondere die Alkalimetall- oder Erdalkalimetallsalze, C₁-C₁₀-Alkanole, wie Methanol, Ethanol und Isopropanol, Zucker, wie Aldosen und Ketosen in Form von Monosacchariden, Disacchariden und Oligosacchariden, insbesondere Glukose, Fruktose und Laktose, Aldehyde, wie Formaldehyd, Bor-Wasserstoff-Verbindungen, wie Borhydride, Borane, Metallboranate und Borankomplexe, wie Diboran, Natriumborhydrid und Aminoborane, insbesondere Trimethylaminoboran, Hydrazin und Alkylhydrazine, wie Methylhydrazin, Hydrogendithionite und Dithionite, insbesondere Natrium- und Kaliumhydrogendithionit, Natrium-, Kalium- und Zinkdithionite, Hydrogensulfide und Sulfide, insbesondere Natrium- und Kaliumhydrogensulfide, Natrium-Kalium- und Calciumsulfide, Hydroxylamin und Harnstoff, und Mischungen davon.

Die WO 2006/005505 beschreibt Katalysatorformkörper, die sich für den Einsatz in dem erfindungsgemäßen Verfahren besonders eignen. Diese können durch ein Verfahren hergestellt werden, bei dem,
(i) ein oxidisches Material, umfassend Kupferoxid, Aluminiumoxid und mindestens eines der Oxide des Lanthans, Wolframs, Molybdäns, Titans oder Zirkoniums wobei die Oxide des Lanthans und/oder Wolframs bevorzugt sein sollen, bereitgestellt wird,
(ii) dem oxidischen Material pulverförmiges metallisches Kupfer, Kupferblättchen, pulverförmiger Zement oder deren Gemische oder ein Gemisch derselben mit Graphit zugegeben werden kann, und
(iii) das aus (ii) resultierende Gemisch zu einer Katalysatortablette oder einem Katalysatorextrudat mit einem Durchmesser d und/oder einer Höhe h < 6,0 mm, Katalysatorkugeln mit einem Durchmesser d < 6,0 mm oder KatalysatorWabenkörper mit einem Zelldurchmesser rz < 6,0 mm verformt wird.

Von den Oxiden des Lanthans, Wolframs, Molybdäns, Titans oder Zirkoniums ist Lanthanoxid bevorzugt. Die Zusammensetzung des oxidischen Materials ist im Allgemeinen so beschaffen, dass der Anteil an Kupferoxid im Bereich von 40 bis 90 Gew.-%, der Anteil an Oxiden des Lanthans, Wolframs, Molybdäns, Titans oder Zirkoniums im Bereich von 1 bis 59 Gew.-% und der Anteil an Aluminiumoxid im Bereich 1 bis 59 Gew.-%, jeweils bezogen auf das Gesamtgewicht der Summe der oben genannten oxidischen Bestandteile, liegt, wobei diese drei Oxide zusammen mindestens 80 Gew.-% des oxidischen Materials nach Calcinierung darstellen, wobei Zement nicht dem oxidischen Material in obigem Sinne zugerechnet wird.

In einer bevorzugten Ausführung umfasst das oxidische Material
(a) Kupferoxid mit einem Anteil im Bereich von 50 ≤ x ≤ 80 Gew.-%, bevorzugt 55 ≤ x ≤ 75 Gew.-%,
(b) Aluminiumoxid mit einem Anteil im Bereich von 15 ≤ y ≤ 35 Gew.-%, bevorzugt 20 ≤ y ≤ 30 Gew.-%, und
(c) mindestens eines der Oxide des Lanthans, Wolframs, Molybdäns, Titans oder Zirkoniums, bevorzugt des Lanthans und/oder Wolframs, mit einem Anteil im Bereich von 2 ≤ z ≤ 20 Gew.-%, bevorzugt 3 ≤ z ≤ 15 Gew.-%,
jeweils bezogen auf das Gesamtgewicht des oxidischen Materials nach Calcinierung, wobei gilt: 80 ≤ x + y + z ≤ 100, besonders 95 ≤ x + y + z ≤ 100.

Bevorzugt wird als Hydrierkatalysator in Schritt b) ein Katalysator eingesetzt, der ein oxidisches Material der Zusammensetzung (CuO)_{0,6-0,8} (Al₂O₃)_{0,1-0,34} (La₂O₃)_{0,02-0,2} umfasst oder aus einem solchen oxidischen Material besteht. Ein speziell geeigneter Katalysator hat die chemische Zusammensetzung 57 % CuO, 28,5 % Al₂O₃, 9,5 % La₂O₃ und 5 % Cu.

Als inertes Trägermaterial für die erfindungsgemäßen Katalysatoren können praktisch alle Trägermaterialien des Stands der Technik, wie sie vorteilhaft bei der Herstellung von geträgerten Katalysatoren Verwendung finden, beispielsweise SiO₂ (Quarz), Porzellan, Magnesiumoxid, Zinndioxid, Siliciumcarbid, TiO₂ (Rutil, Anatas), Al₂O₃ (Tonerde), Aluminiumsilikat, Steatit (Magnesiumsilikat), Zirkoniumsilikat, Cersilikat oder Mischungen dieser Trägermaterialien, eingesetzt werden. Bevorzugte Trägermaterialien sind Aluminiumoxid und Siliciumdioxid. Als Siliciumdioxid-Trägermaterial können Siliciumdioxid-Materialien unterschiedlicher Herkunft und Herstellung, z. B. pyrogen erzeugte Kieselsäuren oder nasschemisch hergestellte Kieselsäuren, wie Kieselgele, Aerogele oder Fällungskieselsäuren, zur Katalysatorherstellung eingesetzt werden (zur Herstellung der verschiedenen SiO₂-Ausgangsmaterialien siehe: W. Büchner; R. Schliebs; G. Winter; K. H. Büchel: Industrielle Anorganische Chemie; 2. Aufl., S. 532 - 533, VCH Verlagsgesellschaft, Weinheim 1986).

Die Katalysatoren können als Formkörper eingesetzt werden, z. B. in Form von Kugeln, Ringen, Zylindern, Würfeln, Quadern oder anderen geometrischen Körpern. Ungeträgerte Katalysatoren können nach üblichen Verfahren geformt werden, z. B. durch Extrudieren, Tablettieren, etc. Die Form geträgerter Katalysatoren wird durch die Form des Trägers bestimmt. Alternativ dazu kann der Träger vor oder nach dem Aufbringen der katalytisch aktiven Komponente(n) einem Formungsverfahren unterzogen werden. Die Katalysatoren können z. B. in Form von gepressten Zylindern, Tabletten, Pastillen, Wagenrädern, Ringen, Sternen oder Strangpresslingen, wie Vollsträngen, polylobären Strängen, Hohlsträngen und Wabenkörpern oder anderen geometrischen Körpern, eingesetzt werden.

Die Katalysatorteilchen weisen im Allgemeinen einen Mittelwert des (größten) Durchmessers von 0,5 bis 20 mm, vorzugsweise 1 bis 10 mm, auf. Dazu zählen z. B. Katalysatoren in Form von Tabletten, z. B. mit einem Durchmesser von 1 bis 7 mm, vorzugsweise 2 bis 6 mm, und einer Höhe von 3 bis 5 mm, Ringen mit z. B. 4 bis 7 mm, vorzugsweise 5 bis 7 mm, Außendurchmesser, 2 bis 5 mm Höhe und 2 bis 3 mm Lochdurchmesser, oder Strängen unterschiedlicher Länge eines Durchmessers von z. B. 1,0 bis 5 mm. Derartige Formen können auf an sich bekannte Weise durch Tablettierung, Strangpressen oder Extrusion erhalten werden. Dazu können der Katalysatormasse übliche Hilfsmittel, z. B. Gleitmittel, wie Graphit, Polyethylenoxid, Cellulose oder Fettsäuren (wie Stearinsäure), und/oder Formhilfsmittel und Verstärkungsmittel, wie Fasern aus Glas, Asbest oder Siliciumcarbid, zugesetzt werden.

Eine spezielle Ausführungsform geträgerter Katalysatoren sind Schalenkatalysatoren. Schalenkatalysatoren umfassen eine schalenförmig auf einen Träger aufgebrachte katalytische Masse. Sie können in Form von Kugeln, Ringen, Zylindern, Würfeln, Quadern oder anderen geometrischen Körpern vorliegen. Unabhängig von der Art und Zusammensetzung des katalytisch aktiven Materials kann die Bereitstellung von Schalenkatalysatorteilchen prinzipiell erfolgen, indem man den Träger mit einem flüssigen Bindemittel und der katalytisch aktiven Masse in Kontakt bringt, dabei eine Schicht der Masse auf dem Träger aufbringt, und anschließend gegebenenfalls das Bindemittel teilweise entfernt. Dabei wird zur Bereitstellung der Katalysatorteilchen das katalytisch aktive Material bereits in seiner fertigen katalytisch aktiven Form, beispielsweise als calciniertes Mischoxid, aufgebracht. Geeignete Verfahren zur Herstellung von Schalenkatalysatoren sind z. B. in der DE-A-29 09 671 und in der EP-A-714 700 beschrieben. Nach dem letztgenannten Verfahren wird der Träger zunächst mit dem flüssigen Bindemittel befeuchtet, dann durch Inkontaktbringen mit trockener, feinteiliger, aktiver Katalysatormasse an der Oberfläche des befeuchteten Trägerkörpers eine Schicht aktiver Katalysatormasse angeheftet und anschließend gegebenenfalls das flüssige Bindemittel teilweise entfernt. In einer speziellen Ausführung werden die Schritte des Befeuchtens des Trägers, des Inkontaktbringens mit der Katalysatormasse und des Entfernens des flüssigen Bindemittels ein- oder mehrfach wiederholt, bis die gewünschte Schichtdicke des Schalenkatalysators erreicht ist.

Eine weitere spezielle Ausführungsform geträgerter Katalysatoren sind durch Tränkverfahren hergestellte Katalysatoren. Dazu können die katalytisch aktiven Katalysatorkomponenten oder Vorläuferverbindungen davon auf das Trägermaterial aufgetragen werden. Im Allgemeinen werden zum Tränken des Trägermaterials wässrige Salzlösungen der Komponenten, z. B. wässrige Lösungen von deren Halogeniden, Sulfaten, Nitraten, etc. aufgebracht. Die Kupferkomponente kann z. B. auch in Form einer wässrigen Lösung ihrer Aminkomplexsalze, beispielsweise als [Cu(NH₃)₄]SO₄-oder als [Cu(NH₃)₄](NO₃)₂-Lösung, gegebenenfalls in Gegenwart von Natriumcarbonat, auf das Trägermaterial aufgetragen werden. Selbstverständlich können auch andere Kupferaminkomplexe als die beispielhaft genannten mit gleichem Erfolg für die Katalysatorherstellung verwendet werden.

Die Imprägnierung des Trägermaterials mit den Vorläuferverbindungen der katalytisch aktiven Komponenten kann prinzipiell einstufig oder mehrstufig erfolgen. Die Imprägnierung kann in herkömmlichen Tränkvorrichtungen, z. B. Imprägniertrommeln, vorgenommen werden. Nach Trocknung und/oder Calcinierung erhält man dann den fertigen Katalysator. Die Trocknung der getränkten Katalysatorformkörper kann kontinuierlich oder chargenweise, z. B. in Band- oder Hordenöfen, erfolgen. Die Trocknung kann bei Atmosphärendruck oder vermindertem Druck erfolgen. Des Weiteren kann die Trocknung in einem Gasstrom, z. B. einem Luftstrom oder einen Stickstoffstrom, erfolgen. Je nach angewandtem Druck wird die Trocknung im Allgemeinen bei Temperaturen von 50 bis 200 °C, vorzugsweise 80 bis 150 °C durchgeführt. Die Calcinierung des gegebenenfalls zuvor getrockneten Katalysators erfolgt im Allgemeinen bei Temperaturen von 200 bis 800 °C, vorzugsweise 500 bis 700 °C. Die Calcinierung kann, wie die Trocknung, kontinuierlich oder chargenweise, z. B. in Band- oder Hordenöfen, durchgeführt werden. Die Calcinierung kann bei Atmosphärendruck oder vermindertem Druck und/oder in einem Gasstrom erfolgen, z. B. in einem Luft- oder einem Wasserstoffstrom. Eine Vorbehandlung mit Wasserstoff oder Wasserstoff enthaltenden Gasen im Allgemeinen unter Bedingungen, die den Hydrierbedingungen entsprechen, dient der Vorreduzierung/Aktivierung des Hydrierkatalysators. Der Katalysator kann aber auch in situ unter den bei der Hydrierung vorgegebenen Bedingungen, vorzugsweise unter Druck (z. B. bei einem Wasserstoffdruck von etwa 100 bis 325 bar), reduziert werden.

Die Hydrierung in Schritt b) erfolgt vorzugsweise bei einer Temperatur in einem Bereich von 100 bis 320 °C, besonders bevorzugt von 150 bis 270 °C, insbesondere von 180 bis 230 °C.

Die Hydrierung in Schritt b) erfolgt vorzugsweise bei einem Druck in einem Bereich von 100 bis 325 bar, besonders bevorzugt von 150 bis 300 bar, insbesondere von 180 bis 230 bar.

Das Molverhältnis von Wasserstoff zu Fettsäuretriglycerid beträgt vorzugsweise 10 : 1 bis 1000 : 1, besonders bevorzugt 12,5 : 1 bis 500 : 1.

Die Katalysator-Belastung bei kontinuierlicher Fahrweise beträgt vorzugsweise 0,1 bis 1, besonders bevorzugt 0,2 bis 0,5 kg zu hydrierendes Fettsäuretriglycerid / kg (Katalysator) x h.

Der Umsatz, bezogen auf Fettsäuretriglycerid, beträgt vorzugsweise wenigstens 90 %, insbesondere wenigstens 95 %.

Die Selektivität bei der Hydrierung in Schritt b), hinsichtlich Propandiol-1,2 (bezogen auf die Summe aus Glycerin und Hydrierprodukten des Glycerins), beträgt bei dem erfindungsgemäßen Verfahren vorzugsweise wenigstens 85 %, besonders bevorzugt wenigstens 90 %. Vielfach können noch höhere Selektivitäten von bis zu 95 % und darüber erzielt werden.

Die Hydrierung wird vorzugsweise kontinuierlich durchgeführt. Der Hydrieraustrag besteht im Wesentlichen aus Fettalkoholen und Propandiol-1,2. Weitere Bestandteile sind ausgewählt unter Methanol, Ethanol, n-Propanol, Isopropanol, Propandiol-1,3, Glycerin, Ethylenglykol und Wasser.

In einer speziellen Ausführung des erfindungsgemäßen Verfahrens erfolgt die Hydrierung in n hintereinander (in Reihe) geschalteten Hydrierreaktoren, wobei n für eine ganze Zahl von wenigstens 2 steht. Geeignete Werte für n sind 2, 3, 4, 5, 6, 7, 8, 9 und 10. Bevorzugt steht n für 3 bis 6 und insbesondere für 2 oder 3. In dieser Ausführung erfolgt die Hydrierung bevorzugt kontinuierlich.

Die zur Hydrierung in Schritt b) eingesetzten Reaktoren können unabhängig voneinander eine oder mehrere Reaktionszonen innerhalb des Reaktors aufweisen. Bei den Reaktoren kann es sich um gleiche oder verschiedene Reaktoren handeln. Diese können z. B. jeweils gleiche oder verschiedene Vermischungscharakteristiken aufweisen und/oder durch Einbauten ein- oder mehrfach unterteilt sein.

Geeignete druckfeste Reaktoren für die Hydrierung sind dem Fachmann bekannt. Dazu zählen die allgemein üblichen Reaktoren für Gas-Flüssig-Reaktionen, wie z. B. Rohrreaktoren, Rohrbündelreaktoren, Gasumlaufreaktoren, Blasensäulen, Schlaufenapparate, Rührkessel (die auch als Rührkesselkaskaden ausgestaltet sein können), Air-Lift-Reaktoren etc.

Das erfindungsgemäße Verfahren unter Verwendung von heterogenen Katalysatoren kann in Festbett- oder Suspensionsfahrweise durchgeführt werden. Die Festbettfahrweise kann dabei z. B. in Sumpf- oder in Rieselfahrweise durchgeführt werden. Dabei werden die Katalysatoren vorzugsweise als Formkörper eingesetzt, wie sie zuvor beschrieben sind, z. B. in Form von gepressten Zylindern, Tabletten, Pastillen, Wagenrädern, Ringen, Sternen oder Strangpresslingen, wie Vollsträngen, polylobären Strängen, Hohlsträngen, Wabenkörpern, etc.

Bei der Suspensionsfahrweise werden ebenfalls heterogene Katalysatoren eingesetzt. Die heterogenen Katalysatoren werden dabei zumeist in feinverteiltem Zustand eingesetzt und liegen im Reaktionsmedium feinteilig suspendiert vor.

Geeignete heterogene Katalysatoren und Verfahren zu ihrer Herstellung sind die zuvor beschriebenen.

Bei der Hydrierung an einem Festbett wird ein Reaktor eingesetzt, in dessen Innenraum ein Festbett angeordnet ist, durch das das Reaktionsmedium strömt. Das Festbett kann dabei aus einer einzigen oder aus mehreren Schüttungen gebildet sein. Jede Schüttung kann dabei eine oder mehrere Zonen aufweisen, wobei wenigstens eine der Zonen ein als Hydrierkatalysator aktives Material enthält. Jede Zone kann dabei ein oder mehrere verschiedene katalytisch aktive Materialien aufweisen und/oder ein oder mehrere verschiedene inerte Materialien aufweisen. Verschiedene Zonen können jeweils gleiche oder verschiedene Zusammensetzungen aufweisen. Es ist auch möglich, mehrere katalytisch aktive Zonen vorzusehen, die beispielsweise durch inerte Schüttungen voneinander getrennt sind. Die einzelnen Zonen können auch unterschiedliche katalytische Aktivität aufweisen. Dazu können verschiedene katalytisch aktive Materialien eingesetzt werden und/oder wenigstens einer der Zonen ein inertes Material beigemischt werden. Das Reaktionsmedium, das durch das Festbett strömt, enthält erfindungsgemäß mindestens eine flüssige Phase. Das Reaktionsmedium kann auch zusätzlich eine gasförmige Phase enthalten.

Als Reaktoren bei der Hydrierung in Suspension kommen insbesondere Schlaufenapparate, wie Strahlschlaufen oder Propellerschlaufen, Rührkessel, die auch als Rührkesselkaskaden ausgestaltet sein können, Blasensäulen oder Air-Lift-Reaktoren zum Einsatz.

Vorzugsweise erfolgt die kontinuierliche Hydrierung in Schritt b) in wenigstens zwei hintereinander (in Reihe) geschalteten Festbettreaktoren. Die Reaktoren werden vorzugsweise im Gleichstrom betrieben. Die Einspeisung der Zuführströme kann sowohl von oben als auch von unten erfolgen.

Unter den Reaktionsbedingungen der Hydrierung liegen das Fettsäuretriglycerid, die resultierenden Fettalkohole und das resultierende 1,2-Propandiol vorzugsweise in flüssiger Phase vor. Dabei können die Bedingungen so gewählt werden, dass einzelne Komponenten des Reaktionsgemischs für sich genommen unter diesen Bedingungen fest wären. Dies gilt z. b. für den/die Fettalkohol(e).

Die Temperatur bei der Hydrierung in Schritt b) beträgt in allen Reaktoren im Allgemeinen etwa 150 bis 300 °C, insbesondere 180 bis 250 °C.

Gewünschtenfalls können bei einer Hydriervorrichtung aus n Reaktoren wenigstens zwei der Reaktoren (d. h. 2 bis n der Reaktoren) eine voneinander verschiedene Temperatur aufweisen. In einer speziellen Ausführung wird jeder nachgeschaltete Reaktor mit einer höheren Temperatur betrieben als der vorherige Reaktor. Zusätzlich kann jeder der Reaktoren zwei oder mehr Reaktionszonen mit unterschiedlicher Temperatur aufweisen. So kann beispielsweise in einer zweiten Reaktionszone eine andere, vorzugsweise eine höhere, Temperatur als in der ersten Reaktionszone bzw. in jeder nachfolgenden Reaktionszone eine höhere Temperatur als in einer vorhergehenden Reaktionszone eingestellt werden, z. B. um einen möglichst vollständigen Umsatz bei der Hydrierung zu erzielen.

Der Reaktionsdruck in Schritt b) beträgt vorzugsweise in allen Reaktoren im Allgemeinen etwa 150 bis 300 bar, besonders bevorzugt 180 bis 250 bar.

Gewünschtenfalls können bei einer Hydriervorrichtung aus n Reaktoren wenigstens zwei der Reaktoren (d. h. 2 bis n der Reaktoren) einen voneinander verschiedenen Druck aufweisen. In einer speziellen Ausführung wird jeder nachgeschaltete Reaktor mit einem höheren Druck betrieben als der vorherige Reaktor.

Die Einspeisung des für die Hydrierung benötigten Wasserstoffs kann in den ersten und gegebenenfalls zusätzlich in wenigstens einen weiteren Rektor erfolgen. Vorzugsweise erfolgt die Einspeisung von Wasserstoff nur in den ersten Reaktor. Die den Reaktoren zugeführte Wasserstoffmenge ergibt sich aus der in der Hydrierreaktion verbrauchten Wasserstoffmenge und der gegebenenfalls mit dem Abgas ausgetragenen Wasserstoffmenge.

Die Einstellung des in dem jeweiligen Reaktor umgesetzten Fettsäuretriglyceridanteils kann z. B. über das Reaktorvolumen und/oder die Verweilzeit im Reaktor erfolgen. Der Umsatz im ersten Reaktor, bezogen auf Fettsäuretriglycerid, beträgt vorzugsweise wenigstens 60 %, besonders bevorzugt wenigstens 70 %. Der Gesamtumsatz in Schritt b), bezogen auf das Fettsäuretriglycerid, beträgt vorzugsweise wenigstens 97 %, besonders bevorzugt wenigstens 98 %, insbesondere wenigstens 99 %.

Zur Abfuhr der bei der exothermen Hydrierung entstehenden Reaktionswärme kann einer oder können mehrere der Reaktoren mit wenigstens einer Kühlvorrichtung versehen sein. In einer speziellen Ausführung ist wenigstens der erste Reaktor mit einer Kühlvorrichtung versehen. Die Abfuhr der Reaktionswärme kann durch Kühlung eines externen Umlaufstroms oder durch interne Kühlung in wenigstens einem der Reaktoren erfolgen. Zur internen Kühlung können die dafür üblichen Vorrichtungen, im Allgemeinen Hohlkörpermodule, wie Field-Rohre, Rohrschlangen, Wärmetauscherplatten, etc. eingesetzt werden. Alternativ kann die Umsetzung auch in einem gekühlten Rohrbündelreaktor erfolgen.

Vorzugsweise erfolgt die Hydrierung in Schritt b) in n hintereinander geschalteten Hydrierreaktoren, wobei n für eine ganze Zahl von wenigstens zwei steht, und wobei wenigstens ein Reaktor über einen in einem externen Kreislauf geführten Strom aus der Reaktionszone verfügt (externer Umlaufstrom, Flüssigkeitsumlauf, Schlaufenfahrweise). Vorzugsweise steht n dabei für zwei oder drei.

Bevorzugt erfolgt die Hydrierung in Schritt b) in n hintereinander geschalteten Hydrierreaktoren, wobei n vorzugsweise für zwei oder drei steht, und der 1. bis (n-1). Reaktor über einen in einem externen Kreislauf geführten Strom aus der Reaktionszone verfügt.

Bevorzugt erfolgt die Hydrierung in Schritt b) in n hintereinander geschalteten Hydrierreaktoren, wobei n vorzugsweise für zwei oder drei steht, und wobei die Umsetzung in dem n. Reaktor (dem letzten von dem zu hydrierenden Reaktionsgemisch durchströmten Reaktor) adiabatisch durchgeführt wird.

Bevorzugt erfolgt die Hydrierung in Schritt b) in n hintereinander geschalteten Hydrierreaktoren, wobei n vorzugsweise für zwei oder drei steht, und wobei der n. Reaktor im geraden Durchgang betrieben wird.

Sofern das in einem der dem ersten Reaktor nachgeschalteten Reaktoren (d. h. dem 2. bis n. Reaktor) hydrierte Reaktionsgemisch nur noch so geringe Anteile an hydrierbarem Fettsäuretriglycerid aufweist, dass die bei der Reaktion auftretende Wärmetönung nicht ausreicht, die erwünschte Temperatur im Reaktor zu halten, kann auch eine Erwärmung des Reaktors (oder einzelner Reaktionszonen des zweiten Reaktors) erforderlich sein. Diese kann analog der zuvor beschriebenen Abfuhr der Reaktionswärme durch Erwärmung eines externen Umlaufstroms oder durch interne Erwärmung erfolgen. In einer geeigneten Ausführung kann zur Temperierung eines Reaktors die Reaktionswärme aus wenigstens einem der vorherigen Reaktoren verwendet werden.

Des Weiteren kann die dem Reaktionsgemisch entzogene Reaktionswärme zur Erwärmung der Zuführströme der Reaktoren verwendet werden. Dazu kann z. B. der Fettsäuretriglyceridzulaufstrom in den ersten Reaktor zumindest teilweise mit einem externen Umlaufstrom dieses Reaktors gemischt und die vereinigten Ströme dann in den ersten Reaktor geführt werden. Des Weiteren kann bei m = 2 bis n Reaktoren der Zuführstrom aus dem (m-1)-ten Reaktor in dem m-ten Reaktor mit einem Umlaufstrom des m-ten Reaktors gemischt und die vereinigten Ströme dann in den m-ten Reaktor geführt werden. Des Weiteren kann der Fettsäuretriglyceridzulaufstrom und/oder ein anderer Zulaufstrom mit Hilfe eines Wärmetauschers erwärmt werden, der mit entzogener Hydrierwärme betrieben wird.

In einer speziellen Ausgestaltung des Verfahrens wird eine Reaktorkaskade aus n in Reihe geschalteten Reaktoren eingesetzt, wobei die Umsetzung in dem n. (n-ten) Reaktor adiabatisch durchgeführt wird. Dieser Begriff wird im Rahmen der vorliegenden Erfindung im technischen und nicht im physiko-chemischen Sinne verstanden. So erfährt das Reaktionsgemisch beim Strömen durch den zweiten Reaktor auf Grund der exothermen Hydrierungsreaktion in der Regel eine Temperaturerhöhung. Unter adiabatischer Reaktionsführung wird eine Vorgehensweise verstanden, bei der die bei der Hydrierung freiwerdende Wärmemenge von der Reaktionsmischung im Reaktor aufgenommen und keine Kühlung durch Kühlvorrichtungen angewandt wird. Somit wird die Reaktionswärme mit dem Reaktionsgemisch aus dem zweiten Reaktor abgeführt, abgesehen von einem Restanteil, der durch natürliche Wärmeleitung und Wärmeabstrahlung vom Reaktor an die Umgebung abgegeben wird. Vorzugsweise wird der n-te Reaktor im geraden Durchgang betrieben.

In einer bevorzugten Ausführung wird zur Hydrierung in Schritt b) eine zweistufige Reaktorkaskade eingesetzt, wobei der erste Hydrierreaktor über einen in einem externen Kreislauf geführten Strom aus der Reaktionszone verfügt. In einer speziellen Ausgestaltung des Verfahrens wird eine Reaktorkaskade aus zwei in Reihe geschalteten Reaktoren eingesetzt, wobei die Umsetzung in dem dritten Reaktor adiabatisch durchgeführt wird.

In einer weiteren bevorzugten Ausführung wird zur Hydrierung in Schritt b) eine dreistufige Reaktorkaskade eingesetzt, wobei der erste und der zweite Hydrierreaktor über einen in einem externen Kreislauf geführten Strom aus der Reaktionszone verfügen. In einer speziellen Ausgestaltung des Verfahrens wird eine Reaktorkaskade aus drei in Reihe geschalteten Reaktoren eingesetzt, wobei die Umsetzung in dem dritten Reaktor adiabatisch durchgeführt wird.

In einer Ausführungsform kann in wenigstens einem der eingesetzten Reaktoren eine zusätzliche Durchmischung erfolgen. Eine zusätzliche Durchmischung ist insbesondere vorteilhaft, wenn die Hydrierung bei großen Verweilzeiten des Reaktionsgemischs erfolgt. Zur Durchmischung können z. B. die in die Reaktoren eingeführten Ströme eingesetzt werden, indem man diese über geeignete Mischvorrichtungen, wie Düsen, in die jeweiligen Reaktoren einführt. Zur Durchmischung können auch in einem externen Kreislauf geführte Ströme aus dem jeweiligen Reaktor eingesetzt werden.

Zur Vervollständigung der Hydrierung wird dem ersten bis (n-1)ten Reaktor je ein Austrag entnommen, der noch hydrierbares Fettsäuretriglycerid enthält und in den jeweils nachgeschalteten Hydrierreaktor eingespeist. In einer speziellen Ausführung wird der Austrag in einen ersten und einen zweiten Teilstrom aufgetrennt, wobei der erste Teilstrom als Kreisstrom dem Reaktor, dem er entnommen wurde, wieder zugeführt wird und der zweite Teilstrom dem nachfolgenden Reaktor zugeführt wird. Der Austrag kann gelöste oder gasförmige Anteile an Wasserstoff enthalten. In speziellen Ausführung wird der Austrag aus dem ersten bis (n-1)ten Reaktor einem Phasentrennbehälter zugeführt, in eine flüssige und in eine gasförmige Phase getrennt, die flüssige Phase in den ersten und den zweiten Teilstrom getrennt und die Gasphase zumindest teilweise dem nachfolgenden Reaktor separat zugeführt. In einer alternativen Ausführung wird der Austrag aus dem ersten bis (n-1)ten Reaktor einem Phasentrennbehälter zugeführt und in einen ersten flüssigen an Wasserstoff abgereicherten Teilstrom und einen zweiten an Wasserstoff angereicherten Teilstrom auftrennt. Der erste Teilstrom wird dann als Kreisstrom dem Reaktor wieder zugeführt, dem er entnommen wurde und der zweite Teilstrom dem nachfolgenden Reaktor (als fettsäuretriglycerid- und wasserstoffhaltiger Feed) zugeführt wird. In einer weiteren alternativen Ausführung erfolgt die Beschickung des zweiten bis n-ten Reaktors mit Wasserstoff nicht über einen dem vorgeschalteten Reaktor entnommenen wasserstoffhaltigen Feed, sondern mit frischem Wasserstoff über eine separate Zuleitung.

Die zuvor beschriebene Verfahrensvariante eignet sich besonders vorteilhaft zur Steuerung der Reaktionstemperatur und des Wärmeübergangs zwischen Reaktionsmedium, begrenzenden Apparatewänden und Umgebung. Eine weitere Möglichkeit zur Steuerung der Wärmebilanz besteht in der Regelung der Eintrittstemperatur des fettsäuretriglyceridhaltigen Zulaufs. So führt eine tiefere Temperatur des eintretenden Zulaufs in der Regel zu einer verbesserten Abführung der Hydrierwärme. Beim Nachlassen der Katalysatoraktivität kann die Eintrittstemperatur höher gewählt werden, um eine höhere Reaktionsgeschwindigkeit zu erreichen und somit die nachlassende Katalysatoraktivität zu kompensieren. Vorteilhafterweise kann so die Standzeit des eingesetzten Katalysators in der Regel verlängert werden.

### Schritt c)

Aus dem in Schritt b) erhaltenen Hydrierungsprodukt wird wenigstens eine fettalkoholhaltige Fraktion isoliert (= Schritt c).

Im einfachsten Fall kann der Hydrieraustrag ohne ihn einer Aufarbeitung zu unterziehen als fettalkoholhaltige Fraktion isoliert werden. Dies ist der Fall, wenn die übrigen enthaltenen Komponenten sich für die angestrebte Verwendung nicht negativ auswirken.

In der Regel wird der Hydrieraustrag einer Aufarbeitung nach üblichen, dem Fachmann bekannten Verfahren unterzogen.

Dazu eignen sich beispielsweise thermische Verfahren, vorzugsweise destillative Verfahren, Adsorption, lonenaustausch, Membrantrennverfahren, Kristallisation, Extraktion oder eine Kombination von zwei oder mehreren dieser Verfahren. Bevorzugt wird der Hydrieraustrag durch Destillation aufgearbeitet. Dazu eignen sich übliche, dem Fachmann bekannte Destillationsverfahren. Geeignete Vorrichtungen für die destillative Aufarbeitung umfassen Destillationskolonnen, wie Bodenkolonnen, die mit Glocken, Siebplatten, Siebböden, Packungen, Einbauten, Ventilen, Seitenabzügen, etc. versehen sein können. Geeignet sind speziell Trennwandkolonnen, die mit Seitenabzügen, Rückführungen, etc. versehen sein können. Zur Destillation kann eine Kombination aus zwei oder mehr als zwei Destillationskolonnen eingesetzt werden. Geeignet sind weiterhin Verdampfer, wie Dünnschichtverdampfer, Fallfilmverdampfer, Sambay-Verdampfer, etc. und Kombinationen davon.

In einer bevorzugten Ausführungsform des erfindungsgemäßen Verfahrens wird in Schritt c) zusätzlich aus dem Produkt der Hydrierung ein 1,2-Propandiol enthaltender Strom isoliert.

Die Erfindung wird anhand der folgenden, nicht einschränkenden Beispiele näher erläutert.

### Beispiele

### Beispiel 1

### Kontinuierliche Hydrierung von Rapsöl an einem Festbettkatalysator

Als Zuführstrom wurde ein Rapsöl eingesetzt, das nach Verseifung und Analyse über GC/MS-Kopplung folgende Fettsäureverteilung aufwies:

| | |
|---|---|
| Palmitinsäure | 4,5 Flächen-% |
| Stearinsäure | 1,8 Flächen-% |
| Ölsäure | 66,0 Flächen-% |
| Linolsäure | 24,2 Flächen-% |

Mit dieser Verteilung ergibt sich für die Hydrierung eine maximal mögliche Ausbeute an Octadecanol von 87 Gew.-% und an 1,2-Propandiol von 6,7%.

Zur Durchführung des Hydrierverfahrens wurde eine zweistufige Laborreaktorkaskade eingesetzt. Der erste Reaktor hatte ein Katalysatorvolumen von 140 ml und der zweite Reaktor ein Katalysatorvolumen von 100 ml. In beiden Reaktoren wurde ein oxidisches Material, das CuO_{0,6-0,85} (Al₂O₃)_{0,1-0,34} (La₂O₃)_{0,01-0,2} umfasst als Katalysator eingesetzt (1. Reaktor: 182 g, 2. Reaktor: 133 g). Der Katalysator wurde vor der Hydrierreaktion im Wasserstoffstrom reduziert.

Der erste Reaktor verfügte über einen Umlauf und wurde in Rieselfahrweise betrieben, der zweite Reaktor wurde im geraden Durchgang betrieben.

Die kontinuierliche Hydrierung erfolgte über 1400 Stunden. Die Temperatur im ersten Reaktor betrug am Reaktoreingang 205 °C und am Reaktorausgang 220 °C, die Temperatur im zweiten Reaktor betrug konstant 200 °C. Der Wasserstoffdruck betrug in beiden Reaktoren 200 bar. Die Katalysatorbelastung wurde auf 0,18 kgrapsöl / L_{Kat} X h eingestellt. Eine Deaktivierung des Katalysators über den Versuchszeitraum wurde nicht festgestellt.

Unter den oben genannten Bedingungen wurde ein Umsatz von 97 % und eine Ausbeute an Octadecanol von 85 Gew.-% (98 % der Theorie) erzielt.

## Patentansprüche

1. Verfahren zur Herstellung von Fettalkoholen mit 6 bis 40 Kohlenstoffatomen, bei dem man
a) einen Strom bereitstellt, der wenigstes ein Fettsäuretriglycerid enthält, das im Wesentlichen aus Glycerinestern höherer Fettsäuren mit 6 bis 40 Kohlenstoffatomen besteht,
b) den Fettsäuretriglycerid enthaltenden Strom einer Hydrierung in Gegenwart eines kupferhaltigen, heterogenen Katalysators unterzieht, wobei zur Hydrierung in Schritt b) ein Katalysator eingesetzt wird, der aus Kupfer, Aluminium und Lanthan besteht, wobei die Katalysatoren die Metalle in oxidischer Form, reduzierter Form oder einer Kombination davon enthalten können,
c) aus dem in Schritt b) erhaltenen Hydrierungsprodukt wenigstens eine fettalkoholhaltige Fraktion isoliert.

2. Verfahren nach Anspruch 1, wobei in Schritt a) ein Strom bereitgestellt wird, der wenigstens ein natürliches Fett und/oder wenigstens ein natürliches Öl enthält.

3. Verfahren nach einem der vorhergehenden Ansprüche, wobei zur Hydrierung in Schritt b) ein Katalysator eingesetzt wird, der aus einem oxidischen Material der Zusammensetzung (CuO)_{0,6-0,8} (Al₂O₃)_{0,1-0,34} (La₂O₃)_{0,02-0,2} besteht.

4. Verfahren nach einem der vorhergehenden Ansprüche, wobei die Hydrierung in Schritt b) bei einer Temperatur in einem Bereich von 100 bis 320 °C, bevorzugt von 150 bis 270 °C, insbesondere von 180 bis 230 °C, erfolgt.

5. Verfahren nach einem der vorhergehenden Ansprüche, wobei die Hydrierung in Schritt b) bei einem Druck in einem Bereich von 100 bis 325 bar, bevorzugt von 150 bis 300 bar, insbesondere von 180 bis 230 bar, erfolgt.

6. Verfahren nach einem der vorhergehenden Ansprüche, wobei die Hydrierung in Schritt b) kontinuierlich erfolgt.

7. Verfahren nach einem der vorhergehenden Ansprüche, wobei die Hydrierung in Schritt b) in n hintereinander geschalteten Hydrierreaktoren erfolgt, wobei n für eine ganze Zahl von wenigstens zwei steht.

8. Verfahren nach Anspruch 7, wobei n für eine ganze Zahl von zwei oder drei steht.

9. Verfahren nach einem der Ansprüche 7 oder 8, wobei die Hydrierung in Schritt b) in n hintereinander geschalteten Hydrierreaktoren erfolgt, wobei n für eine ganze Zahl von wenigstens zwei steht, und der 1. bis (n-1). Reaktor über einen in einem externen Kreislauf geführten Strom aus der Reaktionszone verfügt.

10. Verfahren nach einem der Ansprüche 7 bis 9, wobei die Hydrierung in Schritt b) in n hintereinander geschalteten Hydrierreaktoren erfolgt, wobei n für eine ganze Zahl von wenigstens zwei steht, und wobei die Umsetzung in dem n. Reaktor adiabatisch durchgeführt wird.

11. Verfahren nach einem der Ansprüche 7 bis 10, wobei die Hydrierung in Schritt b) in n hintereinander geschalteten Hydrierreaktoren erfolgt, wobei n für eine ganze Zahl von wenigstens zwei steht, und wobei der n. Reaktor im geraden Durchgang betrieben wird.

12. Verfahren nach einem der Ansprüche 7 bis 11, wobei die Einspeisung von Wasserstoff nur in den ersten Reaktor erfolgt.

13. Verfahren nach einem der vorhergehenden Ansprüche, bei dem man in Schritt c) zusätzlich aus dem Produkt der Hydrierung einen 1,2-Propandiol enthaltenden Strom isoliert.

## Claims

1. A process for preparing fatty alcohols having 6 to 40 carbon atoms, in which
a) a stream comprising at least one fatty acid triglyceride which consists essentially of glycerol esters of higher fatty acids having 6 to 40 carbon atoms is provided,
b) the stream comprising fatty acid triglyceride is subjected to a hydrogenation in the presence of a heterogeneous copper catalyst, using a catalyst which consists of copper, aluminum and lanthanum for hydrogenation in step b), where the catalysts may comprise the metals in oxidic form, reduced form or a combination thereof,
c) at least one fatty alcohol-containing fraction is isolated from the hydrogenation product obtained in step b).

2. The process according to claim 1, wherein, in step a), a stream comprising at least one natural fat and/or at least one natural oil is provided.

3. The process according to either of the preceding claims, wherein a catalyst which consists of an oxidic material of the composition (CuO)_{0.6-0.8} (Al₂O₃)_{0.1-0.34} (La₂O₃)_{0.02-0.2} is used for hydrogenation in step b).

4. The process according to any of the preceding claims, wherein the hydrogenation in step b) is effected at a temperature within a range from 100 to 320°C, preferably from 150 to 270°C, especially from 180 to 230°C.

5. The process according to any of the preceding claims, wherein the hydrogenation in step b) is effected at a pressure within a range from 100 to 325 bar, preferably from 150 to 300 bar, especially from 180 to 230 bar.

6. The process according to any of the preceding claims, wherein the hydrogenation in step b) is continuous.

7. The process according to any of the preceding claims, wherein the hydrogenation in step b) is effected in n hydrogenation reactors connected in series, where n is an integer of at least two.

8. The process according to claim 7, where n is an integer of two or three.

9. The process according to either of claims 7 and 8, wherein the hydrogenation in step b) is effected in n hydrogenation reactors connected in series, where n is an integer of at least two, and the 1st to (n-1)th reactor possesses a stream from the reaction zone which is connected in an external circulation system.

10. The process according to any of claims 7 to 9, wherein the hydrogenation in step b) is effected in n hydrogenation reactors connected in series, where n is an integer of at least two, and wherein the reaction in the nth reactor is performed adiabatically.

11. The process according to any of claims 7 to 10, wherein the hydrogenation in step b) is effected in n hydrogenation reactors connected in series, where n is an integer of at least two, and wherein the nth reactor is operated in straight pass.

12. The process according to any of claims 7 to 11, wherein hydrogen is fed only into the first reactor.

13. The process according to any of the preceding claims, in which a stream comprising 1,2-propanediol is additionally isolated from the product of the hydrogenation in step c).

## Revendications

1. Procédé de préparation d'alcools gras ayant 6 à 40 atomes de carbone, dans lequel
a) on prépare un courant qui contient au moins un triglycéride d'acide gras, qui pour l'essentiel est constitué d'esters du glycérol d'alcools gras supérieurs ayant 6 à 40 atomes de carbone,
b) on soumet le courant contenant le triglycéride d'acide gras à une hydrogénation en présence d'un catalyseur hétérogène contenant du cuivre, auquel cas on utilise pour l'hydrogénation de l'étape b) un catalyseur qui est constitué de cuivre, d'aluminium et de lanthane, les catalyseurs pouvant contenir les métaux sous forme oxyde, sous forme réduite, ou une combinaison de ceux-ci,
c) on isole au moins une fraction contenant des alcools gras du produit d'hydrogénation obtenu dans l'étape b).

2. Procédé selon la revendication 1, dans lequel, dans l'étape a), on prépare un courant qui contient au moins une graisse naturelle et/ou au moins une huile naturelle.

3. Procédé selon l'une des revendications précédentes, dans lequel, pour l'hydrogénation de l'étape b), on utilise un catalyseur qui est constitué d'un matériau de type oxyde, ayant la composition (CuO)_{0,6-0,8} (Al₂O₃)_{0,1-0,34} (La₂O₃)_{0,02-0,2}.

4. Procédé selon l'une des revendications précédentes, dans lequel l'hydrogénation de l'étape b) est mise en oeuvre à une température comprise dans la plage de 100 à 320°C, de préférence de 150 à 270°C, en particulier de 180 à 230°C.

5. Procédé selon l'une des revendications précédentes, dans lequel l'hydrogénation de l'étape b) est mise en oeuvre sous une pression comprise dans la plage de 100 à 325 bar, de préférence de 150 à 300 bar, en particulier de 180 à 230 bar.

6. Procédé selon l'une des revendications précédentes, dans lequel l'hydrogénation de l'étape b) est mise en oeuvre en continu.

7. Procédé selon l'une des revendications précédentes, dans lequel l'hydrogénation de l'étape b) est mise en oeuvre dans n réacteurs d'hydrogénation montés les uns derrière les autres, n représentant un nombre entier valant au moins deux.

8. Procédé selon la revendication 7, dans lequel n est un nombre entier valant deux ou trois.

9. Procédé selon l'une des revendications 7 ou 8, dans lequel l'hydrogénation de l'étape b) est mise en oeuvre dans n réacteurs d'hydrogénation montés les uns derrière les autres, n représentant un nombre entier valant au moins deux, et les réacteurs allant du 1er au (n-1)-ième disposant d'un courant, circulant dans un circuit fermé externe, provenant de la zone de réaction.

10. Procédé selon l'une des revendications 7 à 9, dans lequel l'hydrogénation de l'étape b) est mise en oeuvre dans n réacteurs d'hydrogénation montés les uns derrière les autres, n représentant un nombre entier valant au moins deux, et la réaction dans le n-ième réacteur étant réalisée dans des conditions adiabatiques.

11. Procédé selon l'une des revendications 7 à 10, dans lequel l'hydrogénation de l'étape b) a lieu dans n réacteurs d'hydrogénation montés les uns derrière les autres, n représentant un nombre entier valant au moins deux, et le n-ième réacteur étant exploité par passage direct.

12. Procédé selon l'une des revendications 7 à 11, dans lequel l'injection d'hydrogène n'est effectuée que dans le premier réacteur.

13. Procédé selon l'une des revendications précédentes, dans lequel, dans l'étape c), on isole en outre du produit de l'hydrogénation un courant contenant du 1,2-propanediol.
